# EUROPEAN PATENT SPECIFICATION

(11) **EP 0 518 767 B1**
(45) Date of publication and mention of the grant of the patent: **13.03.2002**
(21) Application number: 92401618.1
(22) Date of filing: 11.06.1992
(51) Int. Cl.: C07D 471/18, C07D 491/22, C07D 519/00, C07D 497/22, A61K 31/435

(54) **Derivatives of amide analogs of certain methano bridged quinolizines**
Amid-Analoge-Derivate von bestimmten methanoverbrückten Chinolizinen
Dérivés d'analogues amide de certaines quinolizines méthano-pontés

(30) Priority: 11.06.1991 EP 91401550
(43) Date of publication of application: 16.12.1992
(73) Proprietor: MERRELL PHARMACEUTICALS INC., Cincinnati, Ohio 45215-6300 (US)
(72) Inventor: Gittos, Maurice W., F-67115 Plobsheim (FR)
(74) Representative: Gillard, Marie-Louise

(56) References cited:
- EP-A- 0 200 444
- EP-A- 0 330 824
- EP-A- 0 377 967
- GB-A- 219 363

## Description

This invention relates to novel amide derivatives of certain 2,6-methano-2H-quinolizines-type compounds, to the intermediates and processes for their preparation, to their ability to induce a blockage of 5HT₃ receptors, and to their end-use application in the treatment of chemotherapeutically-induced nausea and vomiting, as anti-anxiety agents, in the symptomatic treatment of pain associated with migraine, as anti-arrhythmic agents, in the treatment of cognitive disorders, in treating hallucinatory endogenous psychoses of the type manifested in patients suffering from schizophrenia, and mania, in the treatment of glaucoma, for stimulating gastric motility, to combat drug abuse, to treat sleep apnea and to treat irritable bowel syndrome.

More specifically, this invention relates to compounds of the formula 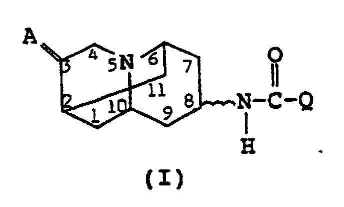 their hydrates, tautomers, stereo and geometric isomers, and to the pharmaceutically acceptable acid addition and quaternary ammonium salts thereof, wherein
- A: is =H₂, =O, =(H)(OH), or 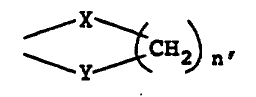 with n being 2 or 3,
X and Y are 0 or S,
- R₁: is H or C₁₋₄alkyl,
- R₂: is H, halogeno, C₁₋₄ alkyl, C₁₋₄ alkoxy, C₃-C₆ cycloalkylmethoxy, OH, -CN or C(O)NH₂,
- Z: is 0, S or NR₁,
- Q: is a heterocyclic moiety of the formulae

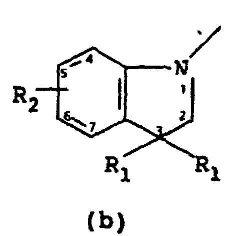

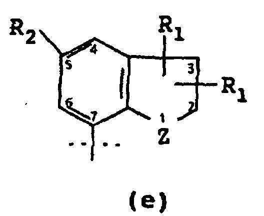

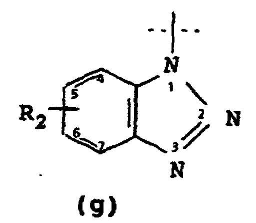

As used herein, the wavy line bonding the nitrogen atom of the 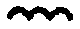 NHC(O)Q moiety to the 8-position of the octa-hydro-2,6-methano-2H-quinolizin moiety (hereinafter sometimes referred to as the methano bridged quinolizinyl moiety) indicates that the bonding may be in the endo (trans) or the exo (cis) configuration. When the wavy line is modified to a solid line it designates the endo configuration while a dotted line indicates an exo configuration. In those instances wherein A represents =(H)(OH) a chirality exists presenting d- or l- isomers and racemic mixtures thereof. The hydrates are those compounds wherein A represents =(OH)(OH). In those instances wherein A represents 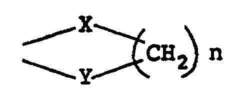 the moiety represents a 5- to 6- membered ring moiety, which, for convenience, may be written -O(CH₂)ₙO-, -O(CH₂)ₙS-, -S(CH₂)ₙS-; the so-indicated terminal bonds of the sulfur and/or oxygen atom being attached to the 3-position carbon atom of the methano bridged quinolizinyl moiety. The C₁₋₄ alkyl radicals include the straight and branched-chain saturated lower aliphatic hydrocarbons having 1 to 4 carbon atoms such as, for example, methyl, ethyl, propyl, isopropyl, n-butyl, and isobutyl; the C₁₋₄ alkoxy radicals are the ether analogs thereof, such as, for example, methoxy and ethoxy. Halogeno includes fluoro, chloro or bromo. Except when otherwise indicated the R₂ substituents attached to the depicted moieties may be attached to any of the carbon atoms of the moiety through which the R₂- bond is depicted. Again for convenience and to avoid any ambiguity, it is to be noted that the valence bond traversing the dotted line is the bond through which the depicted cyclic Q moities of sub-formulae (b), (e) and (g) are attached to the carbon atom of the amide function at the 8-position of the methano bridged quinolizinyl moiety. The R₁ substituents on the Q = (e) moiety may be at the 3,2; 3,3; or 2,2 positions. Preferably, R₁ substituents are at the 3,3 position. The (e) moiety may also have a double bond between positions 2 and 3 with R₁ substituents at appropriate available positions.

Exemplary of the preferred Q heterocycles of sub-formulae (b), (e) and (g) are such moieties as
(b) R₂-substituted-3R₁,3R₁-2,3-dihydro-1H indoles,
(e) 5-R₂-3R₁,3R₁-2,3-dihydro-benzothiophenes and 5-R₂-3R₁,3R₁-2,3-dihydro-benzofurans, and
(g) R₂ substituted-lH-benzotriazoles.

The Q heterocycles of subformulae (b), and (e), are preferred.

The pharmaceutically acceptable acid addition salts referred to above can be non-toxic salts with suitable acids such as those with inorganic acids, for example, hydrochloric, hydrobromic, nitric, sulfuric or phosphoric acids; or with organic acids such as organic carboxylic acids, for example, acetic, propionic, glycolic, maleic, hydroxymaleic, malic, tartaric, citric, salicylic, 2-acetyloxybenzoic, nicotinic or isonicotinic; or organic sulfonic acids, for example, methanesulfonic, ethanesulfonic, 2-hydroxyethanesulfonic, 4-toluenesulfonic or 2-naphthalenesulfonic. Quaternary ammonium salts are formed with alkyl halides such as methyl chloride, methyl bromide, methyl iodide or ethyl bromide; or with sulfate esters such as methyl 4-toluenesulfonate or methyl 2-naphthalene-sulfonate.

In general, the compounds of this invention may be prepared using processes and techniques analogously known in the art. In essence, the compounds of the present invention (1) can be prepared by reacting a di-amine or a reactive derivative thereof, said di-amine having the formula 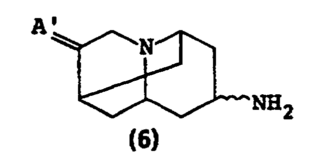 wherein A' is =H₂ or -X-(CH₂)₂-Y-or -X-(CH₂)₃-Y- with X and Y being O or S; with a reactive equivalent of an acid of the formula

QCOOH

wherein Q is defined as above. By a reactive equivalent of the acid is meant the corresponding acid chloride or bromide, the trichloromethyl ester, a mixed anhydride or the carboxylic acid imidazole obtained by the reaction of the appropriate acid halide with N,N-carbonyl diimidazole or any similar acid derivative which would yield the simple carboxylic acid amide on reaction with a primary amine.

The di-amines (6) used as a reactant in the above procedure can be obtained by a multistep procedure from hexahydro-8-hydroxy-2,6-methano-2H-quinolizin-3(4H)one (2) whose synthesis was described in U.S. Patent. 4,906,755. Specifically in the case of the di-amine wherein A' is -O(CH₂)₂O- or -O(CH₂)₃O- the 3,(4H)one is protected as an ethylene or propylene glycol by reaction with ethylene glycol or 1,3-propanediol in the presence of a suitable acid such as p-toluenesulfonic acid in a hydrocarbon solvent such as benzene or toluene using a Dean and Stark apparatus to remove the water. The 8-hydroxy group is then oxidized to a keto group [to produce compounds (4)] by the use of oxalyl chloride and dimethyl sulfoxide following the procedure of Swern and the 8-keto group is transformed into an oxime (compounds 5) by reaction with hydroxylamine hydrochloride. Reduction of the oximino group with a tetrahydrofuran solution of aluminium hydride (generated from lithium aluminium hydride and concentrated sulfuric acid) affords the required di-amine reactant (6) in which the 8-amino group is in the endo (trans) configuration. Reduction of the oximino group by nascent hydrogen generated by dissolving sodium in amyl alcohol affords the di-amine reactant in which the 8-amino group adopts the exo (cis) configuration.

Similarly by using 2-hydroxyethane thiol or 3-hydroxy-propanethiol in the place of ethylene glycol or 1,3-propanediol the above procedure yields the epimeric di-amine wherein A' is -O(CH₂)₂S- and -O(CH₂)₃S- respectively. By using ethylene 1,2-dithiol or propylene-1,3-dithiol in the place of ethylene glycol or 1,3-propanediol the above procedure affords the epimeric di-amine reactants wherein A' is -S(CH₂)₂S- and -S(CH₂)₃S-. These dithianes can be reduced to the epimeric di-amine reactants wherein A' is =H₂ by the use of hydrazine and Raney nickel in a lower alkanol solvent such as 2-propanol at an elevated temperature (60°-100°C).

Various procedures can be used to convert those amides (7) of the invention wherein A' is -X(CH₂)₂Y- or -X(CH₂)₃Y-, X and Y being O or S, to other bridged derivatives of the present invention by standard methods. Thus, the dithioketal, i.e. wherein A' is -S(CH₂)₂S- or -S(CH₂)₃S- can be reduced with hydrazine in the presence of Raney nickel as described above to give the amides of the present invention wherein A" is H₂. The ketals i.e. wherein A' is -O(CH₂)₂O- or -O(CH₂)₃O- can be transformed into those compounds wherein A" is =O by aqueous acid hydrolysis using, for example, 2M HCl at temperatures from 40° to 100°C. The 1,3-oxathiolanes i.e. wherein A' is -O(CH₂)₂S- or -O(CH₂)₃S- can be transformed into the amides wherein A" is =O by reaction with mercuric chloride in aqueous acetonitrile or a lower alcohol at temperatures from 10°-40°C. The keto group can be further transformed into an alcohol (9) group by reduction with an alkali metal borohydride in a lower alcohol. The products from some of these transformations are mixtures of stereo or geometric isomers and these can be separated into their pure isomers by standard methods.

Of course, in those instances wherein the heterocycles moiety of QCOOH contains a primary or secondary amine, it is preferably protected during the above reactions, preferably utilizing a benzyl group when the amine is a secondary amine, and a benzyloxycarbonyl group to protect a primary amine. In either case the protecting group in the product is removed by conventional procedures, e.g., by hydrogenation with H₂ with palladium as a catalyst. Similarly when R₂ functions would interfere with the reactions required for forming the desired product, such moieties may also be protected and then de-protected according to standard techniques and processes well-known in the art.

These reactions may be generically depicted by the following Reaction Scheme. 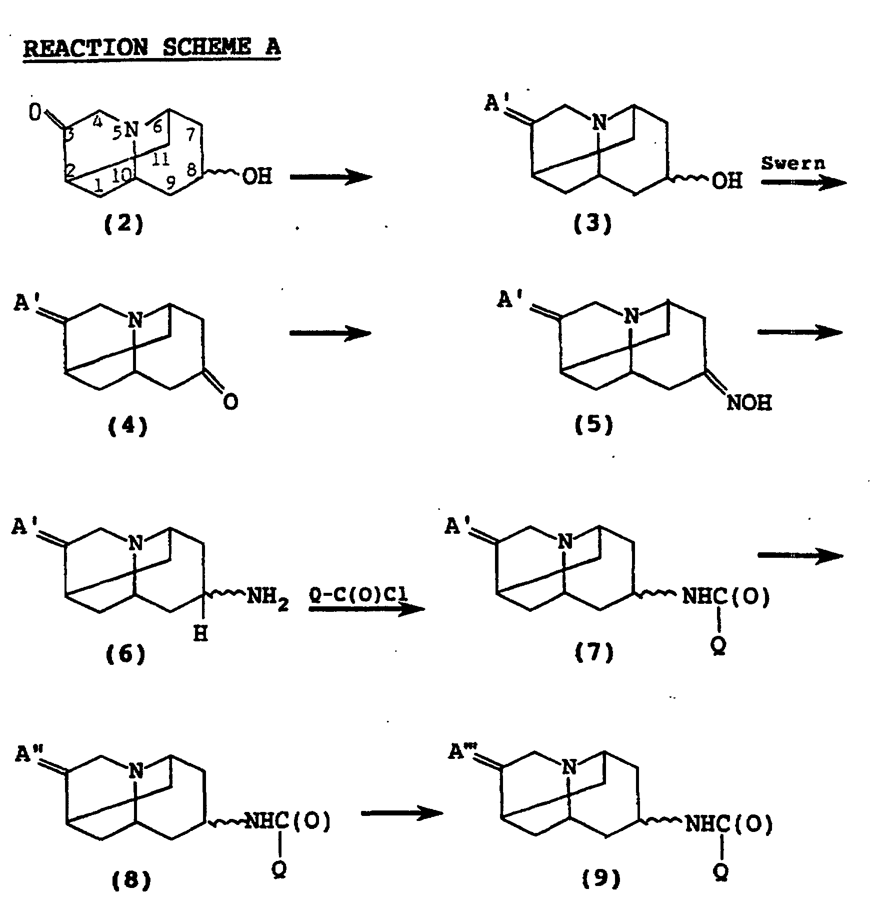 wherein A' is =H₂, -X-(CH₂)₂-Y- or -X-(CH₂)₃-Y-, X and Y being as previously defined as is Q, and A" is O or H₂, and A''' is =(OH) (H).

### EXAMPLE 1

### Trans-octahydrospiro-[1,3-thioxolane-2,3'-(2,6)-methano-2'H-quinolizin]-8'-ol

A stirred mixture of trans-hexahydro-8-hydroxy-2,6--methano-2H-quinolizin-3(4H)-one (9.8 g), methane sulfonic acid (5.88 g), 2-mercapto ethanol (4.35 g) and benzene (200 ml) was refluxed for 2 hours using a Dean and Stark trap. During this time water (1.4ml) was collected. The solid was filtered from the cooled solution, dissolved in water and the solution basified with saturated aqueous potassium carbonate. Extraction with a mixture of ethyl acetate tetrahydrofuran (1:1) and evaporation of the dried extract gave crystals of the titled compound m.p. 194°C (10.4 g).
"Thioxolane" as used herein and hereafter is meant to define the moiety (a) wherein X is oxygen, Y is sulfur and n is 2.

### EXAMPLE 2

### Octahydrospiro-[1,3-thioxolane-2,3'-(2,6)-methano-2'H-quinolizin]-8'-one

A solution of dimethyl sulphoxide (4.07 g) in methylene chloride (20 ml) was slowly added to a stirred solution of oxalyl chloride (6.06 g) in methylene chloride (100 ml) at -70°C under nitrogen. The mixture was stirred for a further 10 minutes and then treated with a suspension of trans-octahydrospiro-[1,3-thioxolane-2,3'-(2,6)-methano-2'H-quinolizin]-8'-ol (10.47 g) in methylene chloride (100 ml). After stirring at -70°C for 15 minutes, triethylamine (30.2 ml) was slowly added and the stirred mixture allowed to return to room temperature overnight. Water (30 ml) and a saturated aqueous solution of potassium carbonate (10 ml) were added and the separated organic phase dried over a mixture of magnesium sulfate and animal charcoal. The residue on evaporation of the solvent was chromatographed on silica gel using a mixture of ethyl acetate and ethanol (4:1) as eluant to give the pure titled compound.

### EXAMPLE 3

### Octahydrospiro-[(1,3)-thioxolane-2,3'-(2,6)-methano-2'H-quinolizin]-8'-one-oxime

A solution of octahydrospiro-[(1,3)-thioxolane-2,3'-(2,6)-methano-2'H-quinolizin]-8'-one (4.5 g) in water (100 ml) was stirred with a solution of hydroxylamine hydrochloride (1.56 g) in water (20 ml) for 1 night at room temperature. The mixture was concentrated, basified by the addition of saturated aqueous potassium bicarbonate and the product extracted with tetrahydrofuran. Some of the solid remained insoluble and was filtered off. The dried organic extracts were evaporated and the combined solid was recrystallized from a mixture of ethanol and methanol to give the titled compound (4.52 g).

### EXAMPLE 4

### Trans-octahydrospiro-[(1,3)-thioxolane-2,3'-(2,6)-methano-2'H-quinolizin]-8'-amine

A solution of concentrated sulfuric acid (3.50 g) in anhydrous tetrahydrofuran was slowly added to a stirred suspension of lithium aluminium hydride (2.72 g) in tetrahydrofuran at -10°C under nitrogen. The mixture was stirred for 2 hours and the temperature allowed to rise to room temperature. Finally powdered octahydrospiro-[(1,3)-thioxolane-2,3'-(2,6)-methano-2'H-quinolizin]-8'-one oxime (4.52 g) then anhydrous tetrahydrofuran (70 ml) were added and the stirred mixture heated at 40°C for 3 hours. After leaving overnight at room temperature the stirred mixture was cooled to 10°C and treated with a solution of water (7 ml) in tetrahydrofuran (7 ml). The mixture was stirred at 30°C for one hour, filtered and the precipitate washed with tetrahydrofuran. The dried filtrate magnesium sulfate was evaporated to give the title compound as a white crystalline solid (3.56 g). The grey precipitate was strongly basified with aqueous potassium hydroxide and the mixture extracted with tetrahydrofuran by decantation. Evaporation of the dried extract gave a further 0.58 g of the white solid. Total yield was 3.74 g (88% yield).

### EXAMPLE 5

### Trans-octahydrospiro-[(1,3)-thioxolane-2,3'-(2,6)-methano-2'H-quinolizin]-8"-yl-2,3-dihydro-3,3-dimethyl-1H-indole-1-carboxamide

A solution of 2,3-dihydro-3,3-dimethyl-1H-indole (2 g) and triethylamine (1.4 g) in methylene chloride (10ml) was added to a stirred solution of trichloromethyl chloroformate (1.64 ml) in methylene chloride at 0°C. The mixture was allowed to stir overnight at room temperature and then washed successively with water (10 ml) and 2N HCl. Evaporation of the dried organic phase gave an oil (2.58 g).

A stirred mixture of the above oil (0.64 g) and trans-octahydrospiro-[(1,3)-thioxolane-2,3'-(2,6)-methano-2'H-quinolizin]-8'-amine (0.5 g) in toluene (25 ml) was refluxed overnight and cooled to room temperature. The solid (0.76 g) was filtered off, partitioned between tetrahydrofuran and saturated aqueous potassium carbonate and the organic phase separated and dried. Evaporation of the organic solvent afforded a solid residue which was purified by silica gel chromatography using methylene chloride methanol (8:2) as eluant, to give the titled compound as a crystalline solid,m.p. 107-108°C.

### EXAMPLE 6

### Trans-2,3-Dihydro-3,3-dimethyl-N-(octahydro-3-oxo-2,6-methano-2H-quinolizin-8-yl)1H-indole-1-carboxamide ethane dioate hydrate

A mixture of trans-octahydrospiro-[(1,3)-thioxolane-2,3'-(2,6)-methano-2'H-quinolizin]-8"-yl-2,3-dihydro-3,3-dimethyl-1H-indole-1-carboxamide (0.33 g), calcium carbonate (0.12 g),mercuric chloride (0.33 g), acetonitrile (16 ml) and water (4 ml) was vigorously stirred at room temperature for two days. The solid was filtered off , washed three times with ethyl acetate and the combined filtrate and washings partitioned between water and a mixture of ethyl acetate-tetrahydrofuran (1:1). The dried organic phase was evaporated and the residue chromatographed on silica gel using firstly ethyl acetate-methanol (4:1) and then methylene chloride-methanol (9:1) as eluants. The base recovered from the second eluant was treated with ethanolic oxalic acid (1 equivalent) to give crystals of the titled compound.

### EXAMPLE 7

### Trans-5-chloro-2,3-dihydro-2,3-dimethyl-N-[octahydrospiro-(1,3-diosolane-2,3'-(2,6)-methano-2'H-quinolizin)-8'-yl]-7-benzofurancarboxamide hydrochloride

A stirred mixture of octahydrospiro-[(1,3)-dioxolane-2,3'(2,6)-methano-2'H-quinolizin]-8'-amine (3.6 g), 5-chloro-2,3-dihydro-2,2-dimethylbenzofuran-7-carboxylic acid chloride (4 g) and toluene (70 ml) was refluxed overnight. After cooling the titled compound was filtered off (6.61 g).

### EXAMPLE 8

### Trans-5-chloro-2,3-dihydro-2,2-dimethyl-N-[octahydro-3-oxo-2,6-methano-2H-quinolizin)-8-yl]-7-benzofurancarboxamide

A stirred solution of trans-5-chloro-2,3-dihydro-2,2-dimethyl-N-(octahydrospiro(1,3-dioxolane-2,3'-(2,6)-methano-2'H-quinolizin)-8'-yl]-7-benzofurancarboxamide hydrochloride (19 g) in 2N HCl (200 ml) was refluxed overnight and allowed to cool. The crystals were filtered off and dried to give the titled compound as its hydrochloride. An aqueous solution of the salt was basified by the addition of saturated aqueous potassium carbonate and the base obtained by extraction with ethyl acetate tetrahydrofuran (1:1) and evaporation of the dried solvent.

### EXAMPLE 9

### Trans-5-chloro-2,3-dihydro-2,2-dimethyl-N-[octahydro-(3-hydroxy-2,6-methano-2H-quinolizin)-8-yl)-7-benzofurancarboxamide methanesulfonate

A mixture of trans-5-chloro-2,3-dihydro-2,2-dimethyl-N-(octahydro-3-oxo-2,6-methano-2H-quinolizin-8-yl)-7-benzo-furancarboxamide (0.35 g), sodium borohydride (0.17 g) and ethanol (15 ml) was stirred overnight at room temperature and the ethanol then evaporated off. A solution of the residue in water was acidified to a pH of 1 by the addition of 2N HCl and the acidic solution basified by the addition of saturated aqueous potassium carbonate. Extraction with ethyl acetate tetrahydrofuran (1:1) and evaporation of the dried extract gave a base which was treated with ethanolic methane sulphonic acid (1 equivalent) to give the titled compound separated with tartaric acid to obtain enantiomers.

### EXAMPLE 10

### Trichloromethyl-1-benzotriazolecarboxylate

A stirred mixture of benzotriazole (3 g), triphosgene (11.22 g), charcoal (1 g) and anhydrous toluene was refluxed overnight and filtered. Evaporation of the filtrate gave an oily solid which was suspended in a mixture of hexane and ether (1:1). Filtration and evaporation of the filtrate gave the titled compound as an oil.

### EXAMPLE 11

### Trans-N-[octahydrospiro(1,3-thioxolane-2,3'-(2,6)-methano-2'H-quinolizin)-8'-yl]-1-benzotriazolecarboxamide

A stirred mixture of trichloromethyl-1-benzotriazolecarboxylate(0.5 g), trans-octahydrospiro-[(1,3)-thioxolane-2,3'-(2,6)-methano-2'H-quinolizin]-8-amine (0.43 g) and toluene (30 ml) was refluxed for 2 hours and cooled. Ether was added and the solid filtered off. A solution of the solid in water was extracted with ethyl acetate and then basified with saturated aqueous potassium carbonate. The mixture was extracted with tetrahydrofuran and the concentrated organic phase chromatographed over silica gel using a mixture of ethyl acetate/methanol (9:1) to give the titled compound as a white solid,m.p. 152°C (0.3 g).

The compounds of the present invention block the M receptors for 5-hydroxytryptamine (5HT) on afferent sensory neurons otherwise known at 5HT₃ receptors. The activity of the compounds against 5HT can be assessed by determining their pA₂ values in the isolated rabbit heart as described by J. R. Fozard et al., Eur. J. Pharmacol. 59, 195-210 (1979). The activity of these compounds against 5HT *in vivo* can be assessed by measurement of the effect of the compound on the Von Bezold-Jarisch reflex induced by 5HT injected intravenously into the rat [see Paintal A. S., Physiol. Rev. 53, 159-227 (1973); J. R. Fozard, Naunyn-Schmiedeberg's Arch. Pharmacol. 326, 36-44 (1984)].

The compounds of this invention may be utilized for their 5HT₃ receptor blocking properties in the treatment of the disease states herein disclosed within the dose range of 0.01 to 10 mg per kilogram of body weight with the preferred compounds being effective within the dose range of 0.01 to 0.1 mg per kilogram of body weight, parenterally, or 0.25 to 1 mg per kilogram of body weight given enterally. The following examples illustrate some methods for testing conditions responsive to treatment with the compounds of the present invention by blocking the 5-HT₃ receptor. Other methods of testing for conditions not illustrated but disclosed herein are well known to those skilled in the art.

As used in this application:
(a) the phrase "gastric motility", refers to the rate at which the stomach empties its contents into the duodenum.
(b) the term "glaucoma" refers to a group of eye diseases characterized by an increase in intraocular pressure, which can cause pathological changes in the optic disk and typically defects in the field of vision.
(c) the term "intraocular pressure" refers to the pressure within the eyeball.
(d) the term "anxiety" refers to a condition where a patient is experiencing fear, apprehension, uncertainty, etc., and can be accompanied with physical manifestations such as, tachycardia, tremors, sweating, etc.
(e) the term "psychosis" refers to a condition where the patient, e.g., a human, experiences a major mental disorder of organic and/or emotional origin characterized by derangement of the personality and loss of contact with reality, often with delusions, hallucinations or illusions, such as, for example, schizophrenia or mania.
(f) the term "treatment" refers to the ability to either relieve or alleviate the patient's disease.
(g) the term patient refers to mammals such as human beings.

The compounds of Formula I exhibit the pharmacological action increasing the motility of the upper gatrointestinal tract. This means that the compounds increase the rate at which the stomach empties its contents into the duodenum.

Thus, the compounds are useful in the treatment of gastric stasis. Gastric stasis refers to a condition where the stomach's ability to empty its contents into the duodenum is impaired. This typically produces discomfort in the patient.

The compounds are also useful in the treatment of gastroesophageal reflux. Gastroesophageal reflux refers to a condition where small quantities of gastric juice are refluxed into the lower part of the esophagus. The acid gastric juice irritates the mucosa of the esophagus causing pain and discomfort in the patient.

The quantity of compound required to produce this gastric motility stimulating effect described above will vary with the particular compound utilized, the patient, the route of administration, the severity of the patient's condition, the presence of other underlying disease states in the patient, and other medications which are being administered concurrently to the patient. Generally though, a patient will respond to dosage range of from 0.01 to 10 mg/kg/day.

One method of demonstrating that the compounds of Formula I increase gastric motility is the following test protocol. Male mice should be fasted overnight prior to being utilized in the test. One group of mice should be administered saline intraperitoneally, and the other group should be administered a compound of Formula I such as at a dose of 5 mg/kg intraperitoneally in a saline carrier.

One hour after administration of either the drug or a saline control, the mice should be given 0.3 ml intragastrically of a suspension containing 10% w/v charcoal, and 5% w/v tragacanth gum with the aid of a feeding needle. Fifteen minutes later the animals should be sacrificed.

The stomachs should be surgically removed and then weighed. The contents should be washed from the stomach, and then the stomachs should be reweighed. The groups should then be compared utilizing the change in weight of the stomach after washing, as an indicator of the rate of gastric emptying.

As noted above, the compounds are also useful as antipsychotics. The quantity of compound required to produce this antipsychotic therapeutic effect will vary with the particular compound utilized, the patient, the severity of the patients illness, the presence of other disease states within the patient, and the mode of administration. Generally though, a patient's psychosis will respond to the compound at a dosage range at from about 0.01 mg/kg to about 10 mg/kg of patient body weight per dosage.

The compounds of Formula I are not dopamine receptor antagonists. Therefore, patients being administered one of these compounds will not experience the numerous side effects that are typically associated with the neuroleptic agents that are currently available, such as chlorpromazine, haloperidol, fluphenazine, etc.

One manner of demonstrating the antipsychotic utility of these compounds is by their ability to block the hyperactivity which usually accompanies the intra-accumbens administration of amphetamine in rats. The following test protocol can be utilized to demonstrate this activity.

This pharmacological effect is measured indirectly. This is accomplished by measuring what effect the compound has upon the ability of a rat to avoid an electrical shock, which it has previously learned to avoid. Initially, the rat should be placed in a test chamber capable of delivering an electrical shock to the rat at a specified rate, for example once every 20 seconds. The test chamber should also be capable of delaying the rate at which electrical shocks are administered if the rat performs the proper avoidance behavior, such as moving from one side of the chamber to the other. The rat should be repeatedly exposed to this test chamber on a regular basis until it has learned to consistently engage in the behavior which delays the response. After it has learned this behavior it is suitable for further testing. A bilateral cannulae should be implanted in the nucleus accumbens according to the following procedure. The rat should be anesthetized and mounted in a stereotactic device. A small hole is drilled through the skull at coordinates A 1.5, L 1.4¹ (relative to bregma) [1 Paximo G. and Watson L., "The Rat Brain in Stereotactic Coordinates", 2^{nd} Ed., m Academic Press, 1986.], bilaterally and an additional hole is drilled near by for a small machine screw. A 20 gauge cannulae is placed stereotactically, so as to terminate 1 mm above, the nucleus (V 6.0, brain surface)¹. Dental acrylic can be utilized to secure the cannulae to the anchor screw and a 25 gauge stylus can be utilized as a plug for each cannulae.

At least seven days after surgery, the rat should be exposed to the electrical stimuli in the test chamber in order to ascertain that it can still engage in the behavior which delays the rate at which shocks are administered. Rats demonstrating this avoidance response are suitable for use in the comparative tests.

The rat should be administered intra-accumbens amphetamine (10 µg/side), subjected to electrical shock in the test chamber and its rate of avoidance recorded.

Thereafter, the rat can be administered the test compound (0.25 ng/side) via the intra-accumbens cannulae. Thirty minutes after administration of the test compound, the rat should be administered intra-accumbens amphetamine (10 mcg/side), subjected to electrical shock in the test chamber and its rate of avoidance recorded.

Rats administered amphetamine alone will exhibit an increased rate of avoidance. Rats administered both amphetamine and a compound of Formula (I) will not exhibit this increased rate avoidance.

The compounds of the present invention exhibit the pharmacological activity of lowering intraocular pressures. Thus, these compounds are useful in the treatment of glaucoma.

The compounds can be administered via ophthalmic dosage forms such as, for example, ophthalmic drops, ophthalmic ointments, and ophthalmic disks. The ophthalmic drops of the present invention should contain from 0.1-10% w/w of one of the compounds of Formula (I). Typically, it will be dissolved in a buffered, isotonic solution containing antimicrobial preservative agents. The ophthalmic ointments will also generally contain from 0.1-10% w/w of one of the compounds of Formula (I) admixed with a suitable base, such as white petrolatum and mineral oil, along with antimicrobial preservatives. The ophthalmic disks will typically be constructed so as to contain a core of active ingredient surrounded by a polymer matrix such as, for example, a hydrophobic ethylene/vinyl acetate copolymer. Specific methods of compounding these dosage forms, as well as appropriate ophthalmic pharmaceutical carriers are known in the art. See Remington's Pharmaceutical Sciences, 16th Ed. Mack Publishing Company, Easton,Pennsylvania (1980).

Typically, the ophthalmic drops or ophthalmic ointments will be administered from 1 to 4 times daily. The ophthalmic disks will be administered weekly.

If desired, the compounds of Formula (I) can be administered systematically in order to lower intraocular pressures. The quantity of compound required to produce this ocular hypotensive effect as the result of systemic administration will vary with the particular compound utilized, the patient, the route of administration, the severity of the patient's glaucoma, the presence of other underlying disease states in the patient, and other medications which are being administered concurrently to the patient. Generally though, a patient's glaucoma will respond to dosage range of from 0.01 to 10 mg/kg/day, if administered systematically.

The compounds of Formula (I) are useful in the treatment of anxiety; that is relieving or alleviating the apprehension, fear, or uncertainty, etc., that patients suffering from anxiety commonly experience, as well as relieving or alleviating the physiological changes associated with anxiety such as tachycardia, tremors, sweating, etc.

The compounds of Formula (I) possess a significant advantage over the anxiolytic agents which are currently available to clinicians, such as chlordiazepoxide, diazepam and other benzodiazepines. The benzodiazepines commonly cause sedation and impairment of motor skills at the dosage levels commonly used in the treatment of anxiety.

The compounds of Formula (I) do not suffer from this disadvantage. They exhibit a wide range at which they demonstrate anxiolytic activity, without causing either sedation or impairment of motor skills.

The quantity of compound required to produce the anxiolytic effect described above will vary with the particular compound utilized, the patient, the route of administration, the severity of the patient's anxiety, the presence of other underlying disease states in the patient, and other medications which are being administered concurrently to the patient. Generally though, a patient's anxiety will respond to dosage range of from 0.01 to 10 mg/kg/day.

The novel compounds of Formula (I) are further useful for the treatment of pain, especially migraine, vascular and cluster headaches and trigeminal neuralgia. They are also useful in the treatment of nausea and vomiting arising from treatment with chemotherapeutic agents, particularly in chemotherapeutic treatment of cancer. The compounds of Formula (I) are also useful in combatting drug-abuse.

In the past, acute attacks of migraine have been treated with a peripheral vasoconstrictor, such as ergotamine, which may be co-administered with caffeine, and dihydroergotamine; an antipyretic analgesic, such as acetylsalicyclic acid or p-acetylaminophenol; and/or an antiemetic such as cyclizine, and thiethylperazine. It has also been reported [J. B. Hughes, Med. J. Aust. 2, No. 17, 580 (1977)] that immediate relief of an acute migraine attack can be obtained by slow intravenous injection of metoclopramide (10 mg).

It is believed that 5-hydroxytryptamine (5-HT) is the naturally accuring substance most likely to play a role in the pathophysiology of migraine. Increased amounts of 5-HT and its metabolite 5-hydroxyindoleacetic acid are excreted in the urine during most attacks. Further, plasma and platelet 5-HT concentrations fall rapidly at the onset of an attack and remain low while the headache persists. Moreover, attacks of migraine have been clearly associated with periods of thrombocytopaenia in certain patients. It has been proposed that compounds which block the activity of 5-HT would be of use in the symptomatic treatment of migraine (J. R. Fozard, International Headache Congress 1980, reported in Advances in Neurology, Vol. 33., Raven Press, New York, 1982).

The known migraine prophylactic drugs, methysergide, propanolol, amitriptyline, and chlorpromazine have widely different pharmacological activities but all are 5-HT D-receptor antagonists at the doses used clinically for the prophylaxis of migraine. Metoclopramide is a 5-HT₃ receptor antagonist and it has been proposed (J. R. Fozard supra) that a blockade of the 5-HT₃ receptor present on afferent sensory neurones affords symptomatic relief in an acute migraine attack.

The potency as 5-HT receptor antagonists of (-) cocaine and some related compounds, including pseudotropyl benzoate (i.e., benzoylpseudotropine) and 3,5-dichlorobenzoyltropine has been reported (J. R. Fozard et al., Eur. J. Pharmacol., 59, (1979) 195-210; J. R. Fozard, Naunyn-Schmiedeberg's Arch Pharmacol., 326, (1984), 36-44. The pA2 values reported for metoclopramide, pseudotropyl benzoate, nor (-) cocaine and benzoyltropine are 7.2, 7.0, 7.7 and 7.2 respectively whilst the pA₂ value determined for 3,5-dichlorobenzoyltropine by the same procedure is 9.3 (J. R. Fozard et al., Eur. J. Pharmacol., 49, (1978) 109-112; J. R. Fozard, Naunyn-Schmiedeberg's Arch Pharmacol., 326, (1984), 36-44. In a double-bind clinical trial, 3,5-dichlorobenzoyltropine proved an effective treatment for the acute migraine attack (C. Loisy et al., Cephalalgia, 5, (1985) 79-82. A further series of tropine esters, with pA₂ values for blockade of the 5-HT M-receptors between 7.7 and 13.6 have been described by Richardson et al., Nature, 316, (1985) 26-131.

In addition, compounds blocking 5-HT₃ receptors, including metoclopramide, 3,5-dichlorobenzoyltropine and (3α-tropanyl)-1H-indole-3-carboxylic acid ester, are highly effective in preventing the nausea and vomiting induced by cancer chemotherapeutic agents in an animal experimental model [W. D. Miner et al., Brit. J. Pharmacol., 88, (1986) 374P; W. D. Miner and G. J. Sanger, Brit. J. Pharmacol., 88, (1986) 497-499; B. Costall et al., Neuropharmacology, 25, (1986) 959-961]. It is believed that cytotoxic drug-induced vomiting involves a 5-HT receptor mechanism [W. D. Miner and G. J. Sanger, Brit. J. Pharmacol., 88, (1986) 497-499]. Accordingly, the compounds of Formula (I) are useful in the treatment of cytotoxic drug-induced vomiting when administered in amounts sufficient to effectively block the said 5-HT₃ receptors.

The dosage range at which the compounds of Formula (I) exhibit their anti-migraine ad anti-emetic effects will vary depending upon the particular compound utilized, the patient, the route of administration, the severity of the patient's condition, the presence of other underlying disease states in the patient, and other medications which are being administered concurrently to the patient. Generally though, a patient's condition will respond to a dosage range of from 0.01 to 10 mg/kg/day.

The compounds of Formula (I) exhibit the pharmacological action in treating sleep apnea. Sleep apnea refers to a condition of transient attacks of failure of automatic control of respiration, resulting in alveolar hypoventilation, which becomes more pronounced during sleep.

One method of demonstrating that the compounds of Formula (1) treat sleep apena is to induce apnea in Wistar rats by i.v. bolus injection of 5-HT or 5-HT agonists and then administer an i.v. injection of the compounds of the present invention. Respiratory parameters and phrenic nerve activity can be monitored. One such procedure is described in the Journal of Pharmacology and Experimental Therapeutics 260(2): 917-924 (1991).

The dosage range at which compounds of Formula (I) exhibit their effect in the treatment of sleep apnea will vary depending upon the particular compound utilized, the patient, the route of administration, the severity of the patients' condition, the presence of other underlying disease states in the patient, and other medications which are being administered concurrently to the patient. Generally though, a patients' condition will respond to a dosage range of from 0.01 to 10 mg/kg/day.

The compounds of Formula (I) exhibit pharmacological activity in treating irritable bowel syndrome. Irritable bowel syndrome is believed to be the consequence of altered colonic motility. Patients complain of constipation or diarrhea and pain. It is believed that 5-HT₃ antagonist may be used to treat irritable bowel syndrome [Gut 31: A1174 (1990); Gastroenterology 98: A394 (1990); Gastroenterology 100: A468 (1991); Gut 32: A1228 (1991)].

The dosage range at which the compounds of Formula (I) exhibit their ability to treat irritable bowel syndrome may vary with the compound used, the patients' condition, the route of administration, etc. Generally though, a patients' condition will respond to a dosage range of from 0.01 to 10 mg/kg/day.

The compounds of Formula (I) can be administered in various manners to achieve the desired effect. The compounds are typically administered either orally or parenterally (subcutaneously, intravenously, intramuscularly). They can also be administered by suppository. As noted above, ophthalmic preparations may also be utilized when glaucoma is being treated.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations. In another embodiment, the compounds of Formula (I) can be tableted with conventional tablet bases such as lactose, sucrose, and cornstarch in combination with binders, such as acacia, cornstarch, or gelatin, disintegrating agents such as potato starch or algenic acid, and a lubricant such as stearic acid or magnesium stearate. Liquid preparations are prepared by dissolving the active ingredient in an aqueous or non-aqueous pharmaceutically acceptable solvent which may also contain suspending agents, sweeting agents, flavoring agents, and preservative agents as are known in the art.

For parenteral administration, the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc. as are known in the art.

The present compounds can be administered in various manners to achieve the desired effect. The compounds can be administered alone or in the form of pharmaceutical preparations to the patient being treated either orally or parenterally, for example, subcutaneously or intravenously. They can also be administered by inhalation or by suppository. The amount of compound administered will vary and can be any effective migraine-relieving amount or amount effective in cytotoxic drug vomiting. Depending upon the patient and the mode of administration, the quantity of compound administered may vary over a wide range to provide from about 0.01 mg/kg to about 10 mg/kg, usually 0.03 to 3.0 mg/kg, of body weight of the patient per dose. Unit doses of these compounds can contain, for example, from about 0.5 mg to 100 mg, usually 1 to 50 mg and preferably 3 to 30 mg, of the compound and may be administered, for example, from 1 to 4 times daily.

The term "unit dosage form" is used herein to mean a single or multiple dose form containing a quantity of the active ingredient in admixture with or otherwise in association with the diluent or carrier, said quantity being such that one or more predetermined units are normally required for a single therapeutic administration. In the case of multiple dose forms such as liquids or scored tablets, said predetermined unit will be one fraction, such as a 5 ml (teaspoon) quantity of a liquid or a half or quarter of a scored tablet, of the multiple dose form.

Specific formulations of the present invention are prepared in a manner well known per se in the pharmaceutical art and usually comprise one or more active compounds of the invention in admixture or otherwise in association with a pharmaceutically acceptable carrier or diluent therefor. The active ingredient will usually be mixed with a carrier, or diluted by a diluent, or enclosed or encapsulated in a capsule, sachet, cachet, paper or other container. A carrier or diluent may be solid, semisolid or liquid material which serves as a vehicle, excipient or medium for the active ingredient. Suitable carriers or diluents are well known per se. See Remington's Pharmaceutical Sciences, Mack Publishing Company, Easton, Pennsylvania, for a description of the preparation of such formulations.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Pat. Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Pat. No. 3,921,636. At least two types of release are possible in these systems. Release by diffusion occurs when the matrix is non-porous. The pharmaceutically effective compound dissolves in and diffuses through the matrix itself. Release by microporous flow occurs when the pharmaceutically effective compound is transported through a liquid phase in the pores of the matrix.

Thus, in summary, the compounds by virtue of their action of blocking 5HT₃ receptors have the end-use applications in the treatment of chemotherapeutically induced nausea and vomiting, as anti-anxiety agents, in the treatment of pain associated with migraine, vascular and cluster headaches and trigeminal neuralgia, in the treatment of arrhythmia, in the treatment of cognitive disorders, psychosis e.g. schizophrenia and for combatting drug abuse, glaucoma, in stimulating gastric motility, to treat sleep apnea and to treat irritable bowel syndrome.

As is generally true for large classes of chemical compounds found to be useful as chemotherapeutic agents, certain sub-classes and certain specific compounds are preferred over others. In the instant application those compounds wherein A represents =H₂, =O or (H)(OH) are preferred, those wherein n is 2 or 3 are preferred, those wherein X and Y are both oxygen or both are sulfur are preferred, those wherein R₁ is H, methyl or ethyl are preferred, those wherein R₂ is H, chloro, bromo, methyl, ethyl, methoxy, OH are preferred, and those wherein Q is a member of the sub-formula (b) are preferred. Specifically preferred compounds are:
trans-5-chloro-2,3-dihydro-N-(octahydro-3-hydroxy-2,6-methano-2H-quinolizin-8-yl)-7-benzofurancarboxamide,
trans-2,3-dihydro-3,3-dimethyl-M-[octahydrospiro[(1,3)-dioxolane-2,3'-(2,6)-methano-2'H-quinolizin]-8'-yl]-1H-indole-1-carboxamide, monohydrochloride,
trans-5-chloro-2,3-dihydro-2,2-dimethyl-N-[octahydrospiro-[1,3-dioxolane-2,3'-(2,6)-methano-2'H-quinolizin]-8'-yl]-7-benzofurancarboxamide, monohydrochloride,
trans-5-chloro-2,3-dihydro-2,2-dimethyl-N-(octahydro-3-oxo-2,6-methano-2H-quinolizin-8-yl)-7-benzofurancarboxamide, monomethanesulfonate,
Trans-octahydrospiro-[(1,3)-thioxolane-2,3'-(2,6)-methano-2'H-quinolizin]-8"-yl-2,3-dihydro-3,3-dimethyl-1H-indole-1-carboxamide,
Trans-N-[octahydrospiro(1,3-thioxolane-2,3'-(2,6)-methano-2'H-quinolizin)-8'-yl]-1-benzotriazolecarboxamide,

## Claims

1. A compound of the formula 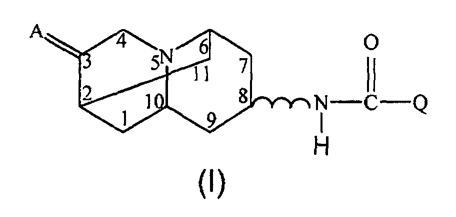 their hydrates, tautomers, stereo and geometric isomers, and the pharmaceutically acceptable acid addition and quaternary ammonium salts thereof, wherein
A is =H₂, =O, =(H)(OH) or 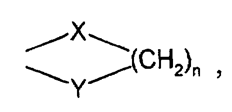 with n being 2 or 3,
X and Y are O or S,
R₁ is H or C₁₋₄ alkyl,
R₂ is H, halogeno, C₁₋₄ alkyl, C₁₋₄ alkoxy, C₃-C₆ cycloalkylmethoxy, OH, -CN or C(O)NH₂,
Z is O, S or NR₁,
Q is a heterocyclic moiety of the formulae 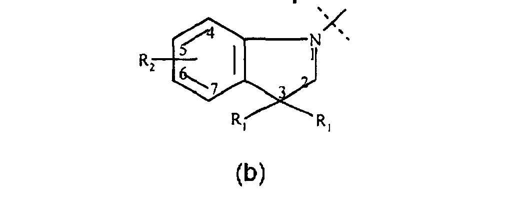 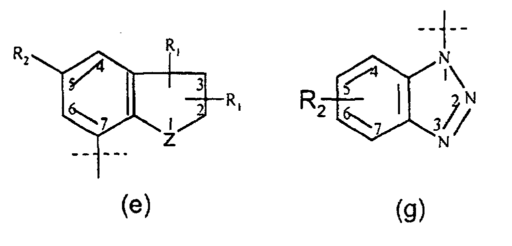
The (e) moiety may also have a double bond between positions 2 and 3 with R₁ substituents at appropriate available positions.

2. The compound of Claim 1 wherein Q is moiety (e).

3. The compound of Claim 1 wherein Q is moiety of formula (e) in which there is a double bond between positions 2 and 3 and R₁ is H.

4. The compound of Claim 1 wherein moiety (e) has both R₁ substituents at position 3.

5. The compound of Claim 1 wherein A is selected from the group consisting of =O, =(H)(OH) or 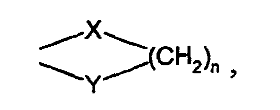 with X and Y each being O.

6. The compound of Claim 1 selected from the group consisting of:
trans-2,3-dihydro-3,3-dimethyl-N-[octahydrospiro [(1,3-dioxolane-2,3'-(2,6)-methano-2'H-quinolizin]8'-yl]-1H-indole-1-carboxamide;
trans-5-chloro-2,3-dihydro-2,2-dimethyl-N-[octahydrospiro[1,3-dioxolane-2,3'-(2,6)-methano-2'H-quinolizin]-8'-yl]-7-benzofurancarboxamide;
trans-2,3-dihydro-3,3-dimethyl-N- (octahydro-3-oxo-2,6-methano-2H-quinolizin-8-yl)-1H-indole-1-carboxamide;
trans-5-chloro-2,3-dihydro-2,2-dimethyl-N-(octahydro-3-oxo-2,6-methano-2H-quinolizin-8-yl)-7-benzofurancarboxamide;
trans-5-chloro-2,3-dihydro-N-[octahydro-3-hydroxy-2,6-methano-2H-quinolizin-8-yl]-7-benzofurancarboxamide;
(-) and (+)-trans-5-chloro-2,3-dihydro-2,2-dimethyl-N-(octahydro-3-hydroxy-2,6-methano-2H-quinolizin-8-yl)-7-benzofurancarboxamide;
Trans-octahydrospiro-[(1,3)-thioxolane-2,3'-(2,6)-methano-2'H-quinolizin]-8"-y]-2,3-dihydro-3,3-dimethyl-1H-indole-1-carboxamide,
Trans-N-[octahydrospiro (1,3-thioxolane-2,3'-(2,6)-methano-2'H-quinolizin)-8'-yl]-1-benzotriazolecarboxamide,

7. A compound of the formula 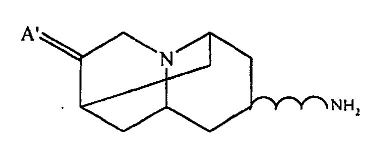 wherein A' is =H₂ or -X-(CH₂)₂-Y- or -X-(CH₂)₃-Y-, each of X and Y being O or S.

8. A method of making a compound of the formula 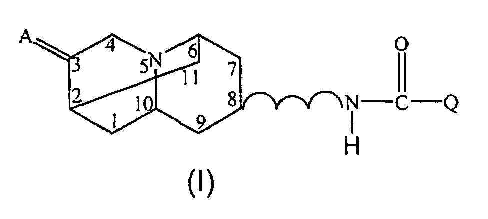 in which A and Q are as defined in Claim 1, by reacting a di-amine or reactive derivative thereof, the diamine having the formula 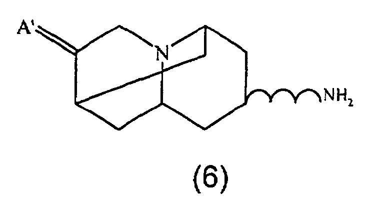 wherein A' is =H₂ or -X-(CH₂)₂-Y- or -X-(CH₂)₃-Y- with X and Y being O or S; with a reactive equivalent of an acid of the formula
QCOOH
Q being as above defined;
and optionally protecting appropriate moieties of Q with appropriate protecting groups during the reaction, and, subsequent to the reaction, optionally removing the protecting groups;
- optionally by reducing the dithioketals, wherein A' is-S(CH₂)₂S- or -S(CH₂)₃S- with hydrazine in the presence of Raney nickel to obtain the compounds of formula (I) in which A = H₂;
- optionally by transforming the ketals wherein A' is -O(CH₂)₂O- or -O(CH₂)₃O- into those compounds wherein A = O by acid hydrolysis;
- optionally by transforming the 1,3-oxathiolanes wherein A' is -O(CH₂)₂S- or -O(CH₂)₃S- into the amides of formula (I) in which A = O by reaction with mercuric chloride in aqueous acetonitrile or a lower alcohol at temperatures of 10°- 40°C;
- optionally by reducing the compounds of formula (I) in which A is =O with an alkali metal borohydride in a lower alcohol for obtaining the compounds of formula (I) in which A is =(H)(OH);
- optionally converting the thus obtained compounds of formula (I) into their pharmaceutically acceptable acid addition salts or quaternary ammonium salts.

9. A compound according to Claim 1 for use as a pharmaceutically active compound.

10. A pharmaceutical composition comprising as active principle a compound according to any one of Claims 1 to 6.

11. A pharmaceutical composition according to Claim 10 wherein the active principle is mixed with a pharmaceutically acceptable carrier.

12. Use of a compound according to Claim 1 for the preparation of a pharmaceutical composition for the treatment of chemotherapeutically induced nausea and vomiting, migraine, arrythmia, cognitive disorders, schizophrenia, mania, glaucoma, gastric motility, drug abuse, sleep apnea and irritable bowel syndrome.

## Patentansprüche

1. Verbindung der Formel 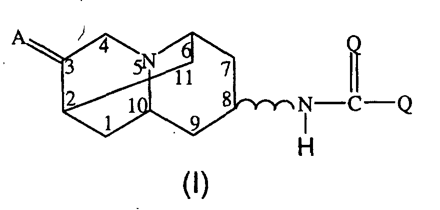 deren Hydrate, Tautomere, Stereo- und geometrischen Isomere und die pharmazeutisch verträglichen Säureadditions- und quartären Ammoniumsalze davon, wobei
A die Bedeutung hat =H₂, =O, =(H)(OH) oder 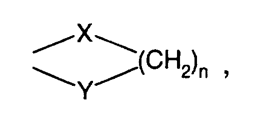 wobei n für 2 oder 3 steht,
X und Y die Bedeutung O oder S haben,
R₁ für H oder einen C₁₋₄-Alkylrest steht,
R₂ für H, ein Halogenatom, einen C₁₋₄-Alkyl-, C₁₋₄-Alkoxy-, C₃-C₆-Cycloalkylmethoxyrest, OH, -CN oder C(O)NH₂ steht,
Z für O, S oder NR₁ steht,
Q eine heterocyclische Einheit der Formeln 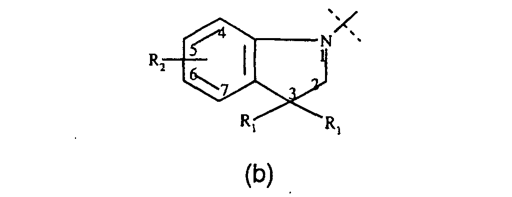 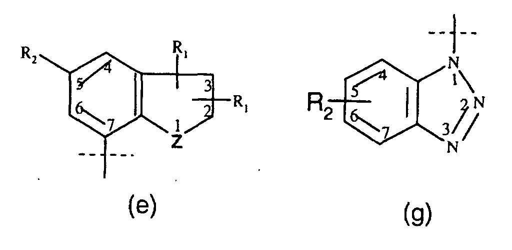
ist. Die Einheit (e) kann auch eine Doppelbindung zwischen den Positionen 2 und 3 aufweisen, wobei R₁-Substituenten an geeigneten, verfügbaren Positionen stehen.

2. Verbindung nach Anspruch 1, wobei Q die Einheit (e) ist.

3. Verbindung nach Anspruch 1, wobei Q die Einheit aus der Formel (e) besteht, wobei eine Doppelbindung zwischen den Positionen 2 und 3 vorliegt und R₁ die Bedeutung H hat.

4. Verbindung nach Anspruch 1, wobei die Einheit (e) beide Substituenten R₁ in der Position 3 trägt.

5. Verbindung nach Anspruch 1, wobei A aus der Gruppe ausgewählt ist, bestehend aus =O, =(H)(OH) oder 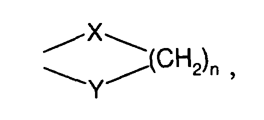 wobei X und Y jeweils die Bedeutung O haben.

6. Verbindung nach Anspruch 1, die aus der Gruppe ausgewählt ist, bestehend aus
Trans-2,3-dihydro-3,3-dimethyl-N-[octahydrospiro[(1,3-dioxolan-2,3'-(2,6)-methano-2'H-chinolizin]-8'-yl]-1H-indol-1-carboxamid;
Trans-5-chlor-2,3-dihydro-2,2-dimethyl-N-[octahydrospiro[1,3-dioxolan-2,3'-(2,6)-methano-2'H-chinolizin]-8'-yl]-7-benzofurancarboxamid;
Trans-2,3-dihydro-3,3-dimethyl-N-(octahydro-3-oxo-2,6-methano-2H-chinolizin-8-yl)-1H-indol-1-carboxamid;
Trans-5-chlor-2,3-dihydro-2,2-dimethyl-N-(octahydro-3-oxo-2,6-methano-2H-chinolizin-8-yl)-7-benzofurancarboxamid;
Trans-5-chlor-2,3-dihydro-N-[octahydro-3-hydroxy-2,6-methano-2H-chinolizin-8-yl]-7-benzofurancarboxamid;
(-)- und (+)-Trans-5-chlor-2,3-dihydro-2,2-dimethyl-N-(octahydro-3-hydroxy-2,6-methano-2H-chinolizin-8-yl)-7-benzofurancarboxamid;
Trans-octahydrospiro-[(1,3)-thioxolan-2,3'-(2,6)-methano-2'H-chinolizin]-8"-yl-2,3-dihydro-3,3-dimethyl- 1H-indol-1-carboxamid;
Trans-N-[octahydrospiro-(1,3-thioxolan-2,3'-(2,6)-methano-2'H-chinolizin)-8'-yl]-1-benzotriazolcarboxamid.

7. Verbindung der Formel 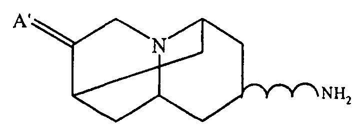 wobei A' die Bedeutung =H₂ oder -X-(CH₂)₂-Y- oder -X-(CH₂)₃-Y- hat, wobei X und Y jeweils für O oder S stehen.

8. Verfahren zur Herstellung einer Verbindung der Formel 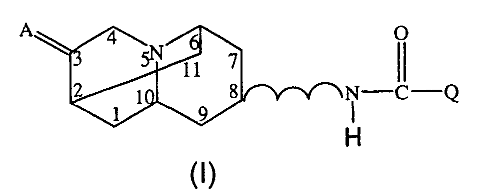 wobei A und Q die im Anspruch 1 definierte Bedeutung haben, durch Umsetzen eines Diamins oder reaktiven Derivates davon, wobei das Diamin die Formel hat 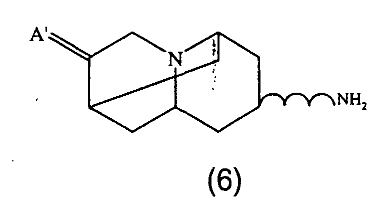 wobei A' die Bedeutung =H₂ oder -X-(CH₂)₂-Y- oder -X-(CH₂)₃-Y- hat, wobei X und Y jeweils für O oder S stehen; mit einem reaktiven Äquivalent einer Säure der Formel
QCOOH
wobei Q die vorstehend definierte Bedeutung hat;
und gegebenenfalls Schützen entsprechender Einheiten Q mit geeigneten Schutzgruppen während der Umsetzung und, nach der Umsetzung, gegebenenfalls Entfemen der Schutzgruppen;
- gegebenenfalls durch Reduzieren der Dithioketale, wobei A' die Bedeutung -S(CH₂)₂S- oder -S(CH₂)₃S- hat, mit Hydrazin in Anwesenheit von Raney-Nickel, so dass die Verbindungen der Formel (I) erhalten werden, in denen A die Bedeutung =H₂ hat;
- gegebenenfalls durch Umwandeln der Ketale, wobei A' die Bedeutung -O(CH₂)₂O- oder -O(CH₂)₃O- hat, in die Verbindungen, in denen A die Bedeutung =O hat, mittels Säurehydrolyse;
- gegebenenfalls durch Umwandeln der 1,3-Oxathiolane, wobei A' die Bedeutung -O(CH₃)₂S- oder -O(CH₂)₃S- hat, in die Amide der Formel (I), in denen A die Bedeutung =O hat, mittels Umsetzen mit Quecksilberchlorid in wässrigem Acetonitril oder einem niederen Alkohol bei Temperaturen von 10°C bis 40°C;
- gegebenenfalls durch Reduzieren der Verbindungen den Formel (I), in denen A die Bedeutung =O hat, mit einem Alkalimetallborhydrid in einem niederen Alkohol, um die Verbindungen der Formel (I) zu erhalten, in denen A die Bedeutung =(H)(OH) hat;
- gegebenenfalls Umwandeln der so erhaltenen Verbindungen der Formel (I) in ihre pharmazeutisch verträglichen Säureadditionssalze oder quartären Ammoniumsalze.

9. Verbindung nach Anspruch 1 zur Verwendung als pharmazeutisch wirksame Verbindung.

10. Arzneimittel, umfassend als Wirkstoff eine Verbindung nach einem der Ansprüche 1 bis 6.

11. Arzneimittel nach Anspruch 10, wobei der Wirkstoff mit einem pharmazeutisch verträglichen Träger gemischt wird.

12. Verwendung einer Verbindung nach Anspruch 1 zur Herstellung eines Arzneimittels zur Behandlung von durch Chemotherapie induziertem Schwindel und Übelkeit, Migräne, Arrhythmie, kognitiven Störungen, Schizophrenie, Manie, Glaukom, Darmbewegung, Drogenmissbrauch, Schlafapnoe und Reizdarmsyndrom.

## Revendications

1. Composé de formule 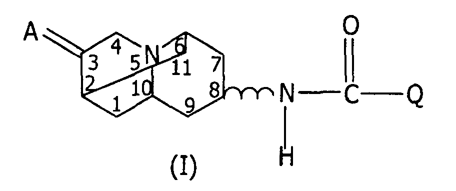 ses hydrates, ses tautomères, ses stéréoisomères et ses isomères géométriques, et ses sels d'addition d'acide et d'ammonium quaternaire pharmaceutiquement acceptables, où
A est H₂, = O, =(H)(OH) ou 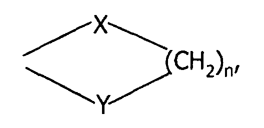 n étant 2 ou 3,
X et Y sont O ou S,
R₁ est H ou alkyle en C₁₋₄,
R₂ est H, halogéno, alkyle en C₁₋₄, alcoxy en C₁₋₄, cycloalkyle en C₃-C₆-méthoxy, OH, -CN ou C(O)NH₂,
Z est O, S ou NR₁,
Q est un groupe hétérocyclique de formules : 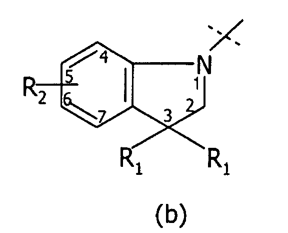 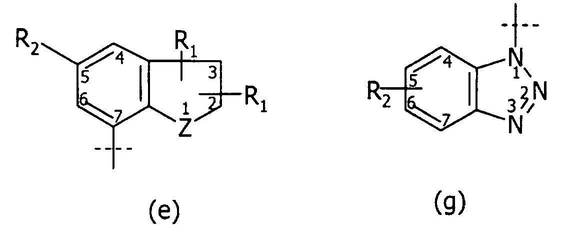
le groupe (e) peut aussi avoir une double liaison entre les positions 2 et 3 avec les substituants R₁ à des positions disponibles appropriées.

2. Composé selon la revendication 1, où Q est le groupe (e).

3. Composé selon la revendication 1, où Q est le groupe de formule (e) où il y a une double liaison entre les positions 2 et 3 et R₁ est H.

4. Composé selon la revendication 1, où le groupe (e) a les deux substituants R₁ en position 3.

5. Composé selon la revendication 1, où A est choisi dans le groupe consistant en =O, =(H)(OH) et 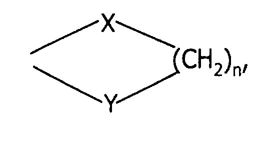 X et Y étant chacun O.

6. Composé selon la revendication 1, choisi dans le groupe consistant en :
le trans-2,3-dihydro-3,3-diméthyl-N-[octahydrospiro[(1,3-dioxolane-2,3'-(2,6)-méthano-2'H-quinolizin]8'-yl]-1H-indole-1-carboxamide ;
le trans-5-chloro-2,3-dihydro-2,2-diméthyl-N-[octahydrospiro-[1,3-dioxolane-2,3'-(2,6)-méthano-2'H-quinolizin]-8'-yl]-7-benzofuranecarboxamide ;
le trans-2,3-dihydro-3,3-diméthyl-N-(octahydro-3-oxo-2,6-méthano-2H-quinolizin-8-yl)-lH-indole-1-carboxamide ;
le trans-5-chloro-2,3-dihydro-2,2-diméthyl-N-(octahydro-3-oxo-2,6-méthano-2H-quinolizin-8-yl)-7-benzofuranecarboxamide ;
le trans-5-chloro-2,3-dihydro-N-[octahydro-3-hydroxy-2,6-méthano-2H-quinolizin-8-yl]-7-benzofuranecarboxamide ;
le (-) et (+)-trans-5-chloro-2,3-dihydro-2,2-diméthyl-N-(octahydro-3-hydroxy-2,6-méthano-2H-quinolizin-8-yl)-7-benzofuranecarboxamide ;
le trans-octahydrospiro-[(1,3)-thioxolane-2,3'-(2,6)-méthano-2'H-quinolizin]-8"-yl]-2,3-dihydro-3,3-diméthyl-1H-indole-1-carboxamide ;
le trans-N-[octahydrospiro(1,3-thioxolane-2,3'-(2,6)-méthano-2'H-quinolizin)-8'-yl]-1-benzotriazolecarboxamide.

7. Composé de formule 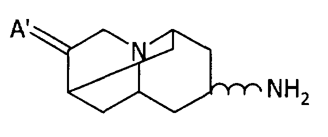 où A' est = H₂ ou -X-(CH₂)₂-Y- ou -X-(CH₂)₃-Y-, X et Y étant chacun O ou S.

8. Procédé de production d'un composé de formule 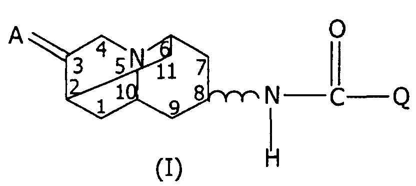 où A et Q sont définis comme dans la revendication 1, par réaction d'une diamine ou d'un dérivé réactif de celle-ci, la diamine ayant la formule 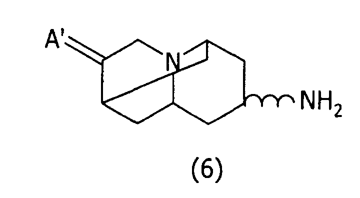 où A' est =H₂ ou -X-(CH₂)₂-Y- ou -X-(CH₂)₃-Y-, X et Y étant O ou S, avec un équivalent réactif d'un acide de formule
QCOOH
Q étant défini comme ci-dessus ;
et éventuellement protection appropriée des groupes Q avec des groupes protecteurs appropriés pendant la réaction, et, après la réaction, éventuellement retrait des groupes protecteurs ;
- éventuellement par réduction des dithiocétals, où A' est -S(CH₂)₂S- ou - S(CH₂)₃S- avec l'hydrazine en présence de nickel de Raney pour obtenir les composés de formule (I) où A = H₂;
- éventuellement par transformation des cétals où A' est -O(CH₂)₂O- ou -O(CH₂)₃O- en les composés où A = O par hydrolyse acide ;
- éventuellement par transformation des 1,3-oxathiolanes où A' est -O(CH₂)₂S- ou -O(CH₂)₃S- en les amides de formule (I) où A = O par réaction avec le chlorure mercurique dans l'acétonitrile aqueux ou un alcool inférieur à des températures de 10°-40°C ;
- éventuellement par réduction des composés de formule (I) où A est = O avec un borohydrure de métal alcalin dans un alcool inférieur pour obtenir les composés de formule (I) où A est =(H)(OH) ;
- éventuellement conversion des composés de formule (I) ainsi obtenus en leurs sels d'addition d'acide ou leurs sels d'ammonium quaternaire pharmaceutiquement acceptables.

9. Composé selon la revendication 1 destiné à être utilisé comme composé pharmaceutiquement actif.

10. Composition pharmaceutique comprenant comme principe actif un composé selon l'une quelconque des revendications 1 à 6.

11. Composition pharmaceutique selon la revendication 10 où le principe actif est mélangé avec un support pharmaceutiquement acceptable.

12. Utilisation d'un composé selon la revendication 1 pour la préparation d'une composition pharmaceutique pour le traitement de la nausée et des vomissements provoqués par une chimiothérapie, de la migraine, de l'arythmie, des troubles cognitifs, de la schizophrénie, des manies, du glaucome, de la motilité gastrique, de l'usage de substances toxiques, des apnées du sommeil et du côlon irritable.
